Europäisches Patentamt

European Patent Office

Office européen des brevets

(11) Publication number: **0 363 203**
**A2**

# EUROPEAN PATENT APPLICATION

(21) Application number: 89310201.2

(51) Int. Cl.⁵: **A61M 1/10** , **A61M 25/10**

(22) Date of filing: 05.10.89

(30) Priority: 05.10.88 US 253663
05.10.88 US 253678
14.10.88 US 257752
18.10.88 US 259056

(43) Date of publication of application:
**11.04.90 Bulletin 90/15**

(84) Designated Contracting States:
**AT BE CH DE ES FR GB GR IT LI LU NL SE**

(71) Applicant: **Abiomed Limited Partnership**
**33 Cherry Hill Drive**
**Danvers Massachusetts 01923(US)**

(72) Inventor: **Milder, Fredric L.**
**204 Clinton Road**
**Brookline Massachusetts 02146(US)**
Inventor: **Moutafis, Timothy E.**
**4 Tucker Street**
**Gloucester Massachusetts 01930(US)**
Inventor: **Stricker, Saul**
**38 Waltham Crescent**
**Richmond Hill Ontario L4B 1Z6(CA)**

(74) Representative: **Hutchins, Michael Richard et al**
**Graham Watt & Co. London Road Riverhead**
**Sevenoaks, Kent TN13 2BN(GB)**

(54) **Cardiac assist balloon and method of insertion.**

(57) A novel cardiac assist balloon assembly and methods of insertion are shown. In one embodiment, the balloon end of the assembly has an intrinsic curvature adapted to the aortic arch, and is straightened by a removable stiff member extending within the catheter to facilitate insertion along the passage. When the balloon is at a curved portion of the passage, the stiff member is retracted, allowing the balloon and adjacent support to conform to the curved passage and advance further without trauma to the vessel. The curved balloon may be stably positioned in a curved arterial passage and pumped with a pulsatile inflation source. In another aspect, an occluding balloon having an asymmetric curvature achieves reliable blood pumping near to the atrium. A torsion-free extender stretches the balloon for compacting it and insertion, and may be inserted over a guide wire, operating without blocking the pressure lumen.

*FIG. 2A*

## CARDIAC ASSIST BALLOON AND METHOD OF INSERTION

### Field of the Invention

This invention relates in general to devices employing balloons mounted on catheters for insertion into patient's arteries to serve upon synchronized inflation and deflation as cardiac assist devices, and more particularly to a specific shape for such a balloon permitting it to operate as a directional cardiac assist device with a single gas lumen in the catheter. The invention also relates to methods and devices for positioning a balloon in the aorta.

### Background of the Invention

The use of catheter supported balloons a devices to assist blood circulation from an ailing heart is now a well recognized medical technique. Such devices are, for example, described in U.S. Patent Application Serial No. 008,061 filed January 14, 1987 entitled High Frequency Intra-Arterial Cardiac Support System which is a Continuation-In-Part of U.S. Serial No. 755,107 filed July 15, 1985 and U.S. Serial No. 063,648 filed June 17, 1987 entitled Intra-Arterial Cardiac Support System. The devices described in those references, as well as other cardiac assist devices discussed in the literature, employ a balloon, which is placed in the aorta outside of the left ventricle. This balloon is pneumatically driven to inflate and deflate on a specific cycle to aid in the pumping of blood from that ventricle into the arterial system.

Some of the devices include valves, which may themselves be additional balloons, for occluding the artery beyond the pumping balloon, both to assist in the pumping and also to prevent high pressure from the artery from entering the portion of the artery occupied by the pumping balloon during the period when it is deflating. In the usual instance such valve balloons are operated by separately controlled air flow from a separate lumen within the catheter. Such devices require not only additional complexity in the structure of the catheter and the inclusion of a second balloon, but also require more complex pneumatic control systems to separately pump the valving balloon and to maintain the specific phase relation required between its action and that of the pumping balloon.

U.S. Letters Patent 3,939,820 describes a single balloon placed in the descending aorta for performing this function. The balloon is formed with a generally cylindrical shape as the pumping element and a larger diameter, disk shaped portion, at the distal end of the balloon. upon inflation the disk shaped portion occludes the aorta before inflation of the cylindrical, pumping portion of the balloon. Thereafter the inflation of the pumping portion of the balloon tends to pressurize the volume within the aorta between the disk shaped occluding portion and the left ventricle valve. This has the beneficial effect of providing a pressurized blood flow to the superior branches from the aorta and to the coronary arteries. In the opposite portion of its cycle, upon deflation of the balloon, the disk shaped portion first collapses, followed by the cylindrical shaped portion. During ventricular ejection however, even the collapsed disk shaped portion presents unwanted resistance to blood flow from the ventricle to the arteries.

In a typical prior art construction, a manifold or handle is mounted to provide a separate fluid connection to each of an inner and an outer tube, which extend for a length of one to one and a half meters from the handle. At the far ends of the tubes a balloon is mounted to be inflated by the outer tube, while the inner tube extends through the balloon to provide a lumen for sampling fluids on the far (cardiac) side of the balloon. Commonly, the balloon is about the size and shape of a long hot dog, although other balloon designs are known.

Insertion of the balloon to a site in the aorta is accomplished by first compacting the uninflated balloon, by folding, wrapping, twisting or the like, and then inserting the compacted balloon assembly through an artery using a guide wire and/or sheath to guide it past irregularities or branches in the artery. Care must be taken during insertion to avoid trauma or perforation, particularly when the balloon is passing branches or curves of the artery.

In a basic variation of the conventional Seldinger technique, such an intraaortic balloon pump (IABP) is inserted via a minor artery by first using a guide wire and dilator to establish a path to the desired location in the aorta, and extending a sheath and dilator along the guide wire to its end. The dilator is removed, leaving the sheath in place. Finally, the folded or wrapped balloon is inserted by pushing its inflation tube through the sheath, thus positioning the balloon at the desired spot. This insertion procedure requires that the balloon occupy a relatively small space.

A number of constructions have been proposed in which a balloon is compacted by inserting a special wrapping wire through the catheter to engage the tip of the balloon, and the wire is turned to turn the balloon tip with respect to the cathether. This twists the balloon around the wrapping wire or a central balloon supporting member. Constructions of a more tangential nature are also known in the field of balloon catheterization, wherein a highly extensible balloon is inflated within an artery to widen the arterial passage. Unlike the balloon of an IABP, the highly elastic balloons used for this purpose generally have a relaxed or uninflated state which is already highly compact, so that insertion mechanisms are not clearly analogous. Examples of some of the foregoing constructions are shown in U.S. Patents 4,362,150 of Lombardi et al.; 4,531,512 of Wolvek et al.; 4,311,133 of Robinson and 4,292,974 of Fogarty. Other constructions provide a rotatable support member about which the balloon is rolled or twisted, possibly with the aid of an external balloon-restraining or engaging member, to wrap the balloon.

Prior art cardiac assist balloons are fabricated of a pre-shaped thin membrane of low intrinsic stiffness, so it is customary to provide a support member within the balloon to hold the balloon extended in the longitudinal sense during pumping. This support member, which may simply be the end portion of the inner tube, or which may include a separate wire or wire-wrapped reinforcement, limits the ability of the balloon to follow or conform to a curved arterial passage, and does not permit the balloon to remain stably positioned in a curved arterial section during pumping.

Objects and Summary of the Invention

It is an object of the invention to provide a cardiac assist balloon, and a method and apparatus for facilitating insertion of such a balloon.

It is another object of the invention to provide a method and apparatus for insertion of a balloon having a relatively large diameter or irregular shape, or for insertion of a balloon along a bent or tortuous path.

It is another object of the invention to provide a single balloon device having an inflated shape such that it serves both as an occluding valve in the artery and also as a pumping member to provide cardiac assist, while in the deflated state it is substantially completely collapsed and provides minimal resistance to the blood flow from the ventricle to the artery.

It is another object of the invention to provide an insertable arterial balloon assembly having a curved balloon support structure which may be selectively straightened or stiffened, or relaxed and curved, during insertion.

It is another object of the invention to provide a balloon structure which is stably positioned in a curved arterial passage during pumping.

Broadly speaking, an inflatable balloon for the preferred practice of the present invention is formed of a thin elastomeric material. The balloon is generally elongated along a first axis which may be either straight or curvilinear and has its maximum dimension in a plane transverse to the first axis at a point intermediate the ends of the balloon along the first axis. This maximum dimension is larger than the inner diameter of the artery (typically the ascending aorta) in which it is placed. The balloon is provided with an orifice at one end for attachment to a catheter and is tapered either symmetrically or asymmetrically from the point of maximum dimension toward the tip end opposite the orifice to a dimension substantially reduced from the maximum dimension. It is also tapered in the opposite direction toward the orifice end with a generally symmetrical taper. If the taper is asymmetrical then the orifice or the tip end or both is located off axis.

In the inflation process the wider dimensional portion of the balloon inflates first, occluding the artery. As the inflation process continues the forward tapered portion of the balloon further inflates, increasing the pressure of blood in the volume between the occluding point and the heart valve.

In the deflated condition, the tapers toward the tip and orifice ends provide that the balloon collapses so as to present a minimal profile opposing the flow of blood past the balloon.

Such an occluding balloon, when sized to operate in the ascending aorta, poses problems of insertion and stable positioning which are addressed according to other aspects of the invention.

According to one such aspect, an arterial balloon assembly has an intrinsically curved structure at the balloon end. A straightening member is inserted through the inflation manifold to the balloon for causing the assembly to straighten out. In this configuration the assembly is adapted for insertion through an artery; when the straightening member is drawn back a few centimeters the balloon end re-assumes its predetermined intrinsic flexibility and curvature. In the relaxed or curved configuration, the balloon may lie quiescent in, or be advanced to a curved arterial path. In a preferred embodiment, the curvature conforms to the sharply curved passage between the ascending and descending aorta. During pumping, the curvature eliminates creeping of the balloon characteristic of a straight balloon structure.

According to another aspect, an arterial balloon assembly includes a mechanism for extending a balloon to a stretched state prior to insertion. The mechanism includes an extender member which extends through a catheter from the handle to the balloon, and which advances to extend the balloon significantly while twisting it at most a relatively small amount. An actuation assembly is comprised of one part, preferably a grooved sleeve, in the handle, and a cooperating part, preferably a shank, affixed to the extending member and having a mating protrusion sliding in the groove. This defines a precisely aligned and controlled balloon extension for compacting the balloon when the extender member is advanced. The mechanism permits the balloon to be fully relaxed once it is inserted, and to be recompacted, following use, for removal from the patient.

In yet another aspect, an arterial balloon assembly includes a mechanism for advancing and twisting a balloon to a stretched and tightly wrapped state prior to insertion. The mechanism includes a torquing member which extends through a catheter from the handle to the balloon, and which advances to extend the balloon significantly while twisting it a relatively small amount. A screw assembly comprised of one part, preferably a nut, in the handle, and a cooperating part, preferably a mating threaded shank, affixed to the torquing member, turns the member. This defines a precise ratio of balloon extension to twist, and also defines fixed total amounts of twist and extension of the torquing member.

These mechanisms are of particular utility for a large diameter balloon, or for a balloon having a non-cylindrical side wall or one which deviates substantially from straightness, for example, one having a polygonal or highly curved contour. It is of particular utility for inserting a balloon along an arterial path having a narrow passage or a sharp curve.

Brief Description of Drawings

These and other features of the invention will be understood with reference to the description herein of presently preferred embodiments, taken together with the drawings, wherein:

Figure 1 shows a prior art balloon catheter;

Figures 2A, 2B show cross-sectional views of one embodiment of a balloon catheter and insertion mechanism according to the invention;

Figures 2C, 2D show cross-sectional views of another embodiment of a balloon and insertion mechanism according to the invention;

Figures 3 and 3A, 3B, and 3C show a schematic view and detailed sectional views illustrating different aspects of the invention, and variations in a balloon tip construction;

Figure 4 is a detailed cross-sectional view of a handle portion used with the embodiment of Figures 2A, 28;

Figure 4A is a detailed, cross-sectional view of a handle portion used with the embodiment of Figures 2C, 2D;

Figure 5 is a perspective view of an extension member used in the practice of the invention;

Figures 6A, 6B show views of another embodiment of a stretch mechanism according to the invention;

Figures 7A, 7B illustrate another embodiment of a balloon extension mechanism;

Figure 8 is an illustration in perspective view of a preferred catheter balloon constructed in accordance with the principles of this invention for placement in the ascending aorta;

Figure 9 is an illustration in cross sectional view of the catheter balloon of Figure 8;

Figure 10 is a geometrical sketch of one embodiment of a catheter balloon constructed in accordance with the principles of this invention;

Figure 11 shows a prior art intraaortic balloon pump;

Figures 12A-12C show corresponding views of an intraaortic balloon pump according to the present invention; and

Figure 13 shows a partial cutaway view of the balloon end of the embodiment of Figures 12A-12C.

Detailed Description of Illustrated Embodiments

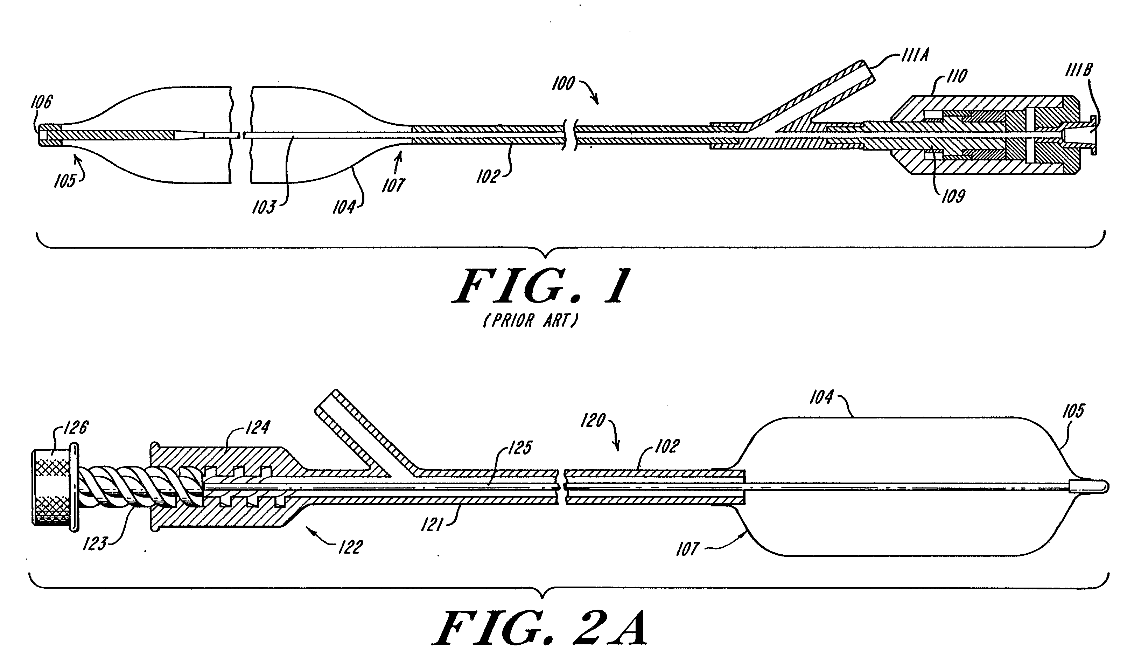

The invention is best understood in relation to the existing art of balloon catheters, of which Figure 1 illustrates an example 100. Such a device consists of a bladder or balloon 104 formed of a thin walled polymer and having a defined shape, which is integrally connected to an inflation tube 102. Balloon 104 is closed at its distal end 105 and communicates via its other end 107 with the tube 102 so that by applying fluid pressure to the lumen of the tube, the balloon may be inflated. The tube 102 is sealed to the proximal end 107 of the balloon and an inner tube or support member 103 extends through the balloon to its distal end 105. The near end of tube 102 is connected to a handle 110 which serves as a manifold with ports 111A, 111b for the provision of fluid to, or the sensing of fluid pressures in, the catheter. Inner member 103 may provide a lumen extending from port 111A to a sensing port 106 at the distal side of the balloon for monitoring fluids at the heart. In the prior art device of Figure 1, which is taken substantially from a description in U.S. Patent 4,362,150, a threaded member 109 in handle 110 moves the inner member with a rotational motion to effect a large number of turns of the balloon with negligible axial motion in order to compact the balloon 104.

Figures 2A, 28 show sectional views of one embodiment of a balloon insertion device 120 according to the present invention. For clarity, the view corresponds to that of Figure 1, and both the balloon and the tube are identified by numerals 104, 102 identical to those used in Figure 1. In this embodiment, a handle portion 121 attached to the tube 102 contains an actuator mechanism 122 which includes a male portion 123 and a female portion 124 which cooperate to move a torquing member 125 extending from the male screw portion to the balloon. According to one aspect of the invention, the ratio of linear to rotational motion is high. For example, the pitch of the mating portions 123, 124 is over five millimeters per turn, and preferably approximately ten millimeters.

Torquing member 125 extends the length of the inflation tube 102, which may be, for example, 1.25 meters, and engages the distal end 105 of the balloon 104. When balloon end 105 is gripped and pushed by the torquing member 125, distal end 105 is stretched away from the proximal end 107 of the balloon, and the body of the balloon is both stretched and slightly twisted. A knurled knob 126 is attached to the end of screw portion 123 and permits the user to rotate that portion for operation. The two figures, 2A, 2B illustrate the same device in two different positions.

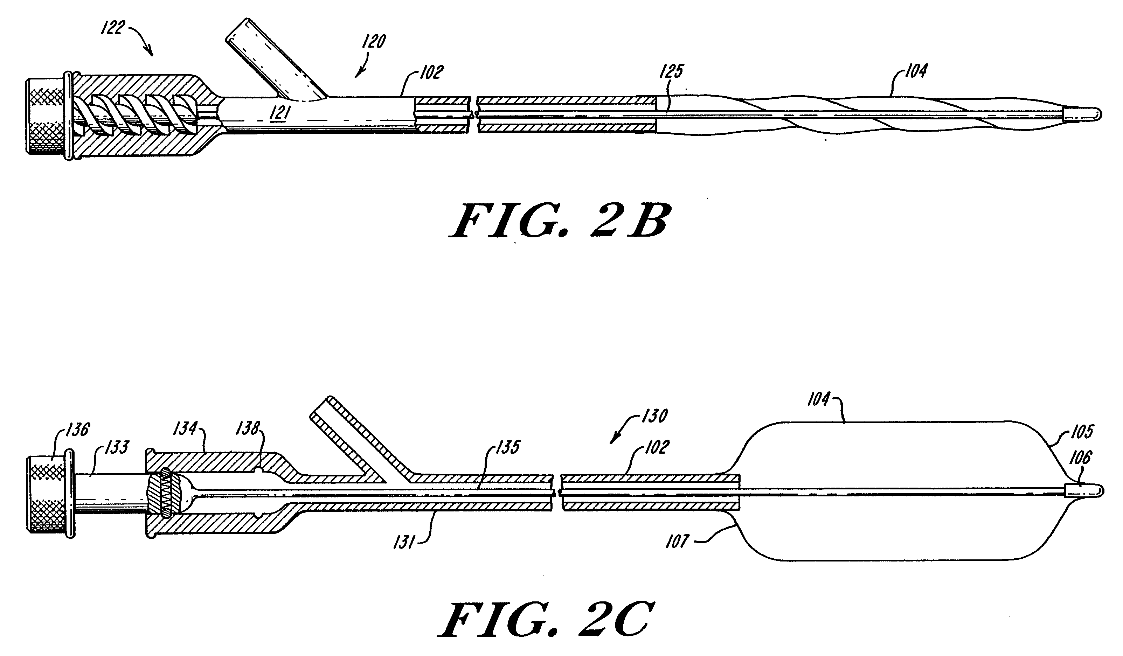

As shown in Figure 2A, the screw actuation mechanism is in its retracted position, so that member 125 does not bear against the balloon, and balloon 104 is in its flaccid, or resting state. In this state, it occupies a relatively large volume, and has a substantially greater diameter than that of the inflation tube 102. As shown in Figure 2B, the actuation mechanism is fully advanced, and the balloon has been compacted. More precisely, the balloon is stretched and twisted to attain a diameter comparable to that of the inflation tube. By way of scale, a prototype balloon was made approximately thirty-five millimeters diameter and seven centimeters in length. The balloon was non-cylindrical in shape and was also formed with a front-to-back asymmetry. The relatively wide and asymmetrical shape made it difficult to compact the balloon sufficiently for insertion using conventional folding or rolling techniques. Using an actuator mechanism as just described having approximately a 25 millimeter total extension with approximately 2 1/2 screw turns, the balloon was compacted by stretching and twisting to attain a diameter roughly equal to that of the inflation tube. In this compacted state the balloon was readily inserted through a sheath of small diameter.

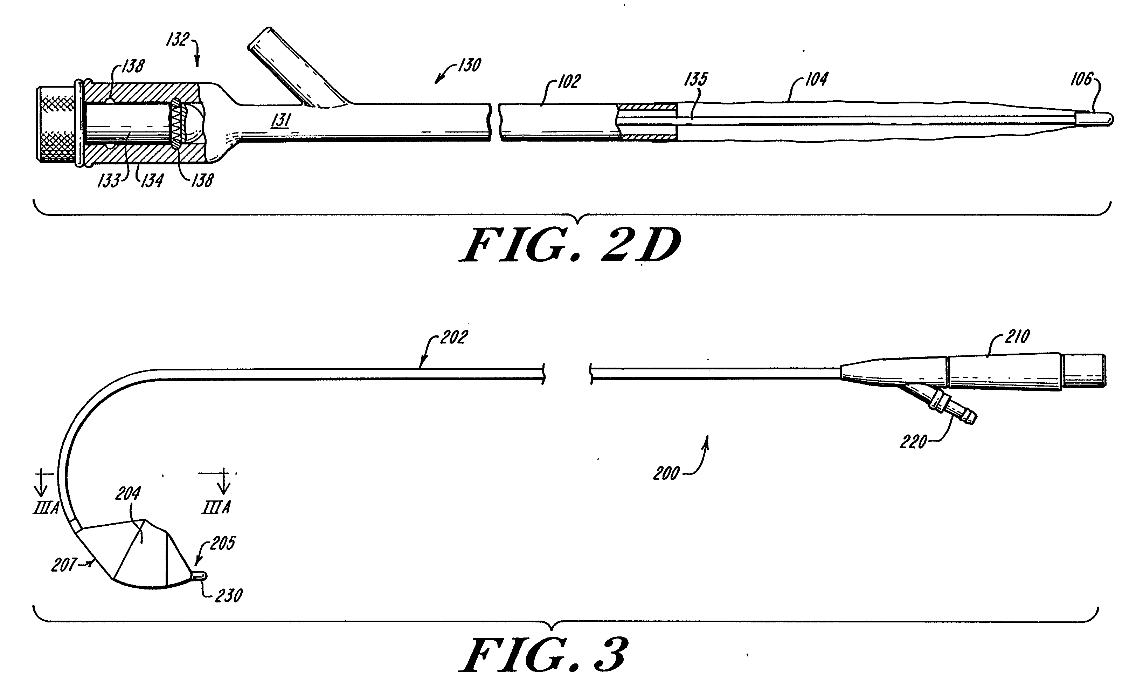

Figures 2C, 2D show partial cutaway views of another embodiment of a balloon insertion device 130 according to the present invention. For clarity, the view corresponds to that of Figures 2A, 2B. In this embodiment, a handle portion 131 attached to the tube 102 contains an actuator mechanism 132 which includes an outer aligning sleeve 134 and an inner extender portion 135 which cooperate to move an extender member 135 which runs from the inner portion to the balloon. In this embodiment of the invention, the sleeve and inner member hold the extender in alignment to provide a purely linear displacement in the range of two to five centimeters; a plurality of notched detent positions 138, of which two are illustrated, provide defined stops at known extensions.

Extender member 135 extends the length of the inflation tube 102, and engages a thrust hub 106 of the distal end 105 of the balloon 104. When balloon end 105 is engaged and pushed by the extender member 135, distal end 105 is pushed away from the proximal end 107 of the balloon, and the body of the balloon is stretched and drawn into a straightened and compact state. Significantly, the rotational orientation of the balloon remains unchanged when extended as the device advances without rotating. A knurled knob 136 is attached to the end of inner extender portion 133 and permits the user to grip that portion for operation. The two figures, 2C, 2D illustrate the same device in two different extensions.

As shown in Figure 2C, the actuation mechanism is in its retracted position, so that extender member 135 does not exert force against the balloon, and balloon 104 is in its flaccid, or resting state. In this state, it occupies a relatively large volume, and has a substantially greater diameter than that of the inflation tube 102. As shown in Figure 2D, the actuation mechanism is fully advanced, and the balloon has been compacted. More precisely, the balloon is stretched by an amount which may be fifteen to seventy-five percent of its resting length, to fit through a diameter comparable to that of the inflation tube.

In each of these embodiments, the extender member 125, 135 preferably is a torsionally-stiff member which either transmits torque for twisting the balloon by a small and precisely controlled amount, or resists torque to prevent twisting of the balloon structure.

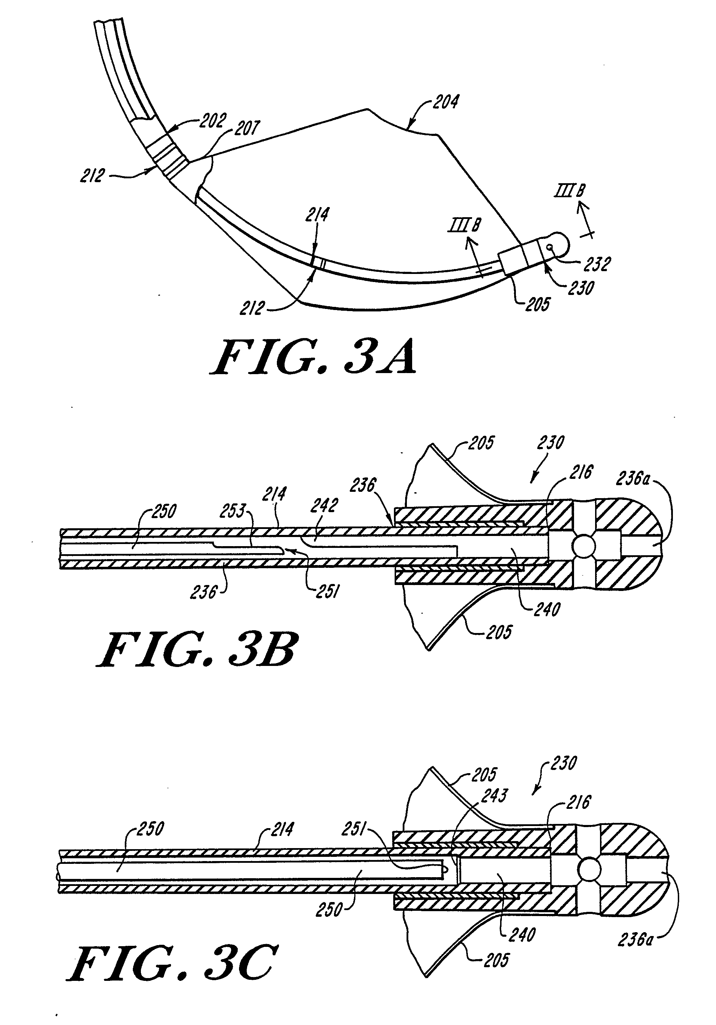

Figure 3 is a schematic representation of another embodiment 200 of the invention, which will be discussed in greater detail below. A balloon 204 is mounted at the end of an inflation tube 202 which extends from a handle 210. The handle constitutes a bifurcation assembly which provides an inflation port 220 interconnected with the balloon 204 via the tube 202, and, as described in greater detail below, an access port for a second tube which communicates with a nosepiece 230 at the distal end of the balloon. The nosepiece, which extends ahead of the balloon, is formed of a strong material, such as polycarbonate. It constitutes a thrust-receiving hub attached to the balloon, like hub 106 of the preceding embodiment, with additional structure for forming a fluid sampling port ahead of the balloon.

Figure 3A is a cutaway view of the balloon of Figure 3, illustrating in further detail internal elements of the balloon and of the set 200. It will be seen that inflation tube 202 seals to the proximal end 207 of the balloon, and that a second or internal tube 214 extends through the inflation tube and connects to the nosepiece 230, which in turn defines an opening or port 232 ahead of the distal end of the balloon. Tantalum markers 212 on each tube 202, 214 permit precise fluoroscopic visualization of the tube end during insertion. Both tubes are made of an appropriate tubing suitable for medical use, such as a polyurethane tubing.

Figure 3B shows one embodiment of the nosepiece 230 in greater detail, connected to the end 205 of the balloon, forming a central hub with one or more holes communicating with a central passageway 236a to provide a fluid access port for the inner lumen of the device. Nosepiece 230 constitutes a rigid structural member for bearing against a separate extender member which is provided in this embodiment to compact the balloon. Within the nosepiece, inner tube 214 butts against an end wall 216 and is permanently cemented thereto. Within the tube 214, a thrust insert 240 also abuts wall 216 and is press-fit to provide rigid engagement with tube 214 and nosepiece 230. This provides an end assembly against which the extender member may be advanced to extend the balloon forwardly.

The illustrated thrust insert 240 includes a segment of stainless steel tubing having an outer tube diameter matched to the inner diameter of tube 214. At the proximal end of insert 240, approximately one-half the diameter of the insert tube material has been removed and the end beveled, forming an engaging finger 242 in the form a partial tubular shell. The engaging finger 242 is adapted to engage a similarly-shaped solid or hollow end of a metal extender member 250 which is inserted through the tube 214 from handle 210, and which is advanced to stretch the balloon as previously described.

The extender member 250 corresponding to member 125 of Figures 2A, 2B or 135 of Figures 2C, 2D is inserted through the inner lumen 236 starting at the handle and extending to the balloon. For clarity of illustration, the distal end 251 of the extender member is shown in Figure 3B in a position axially withdrawn from the thrust insert 240. One edge of end 251 is flattened, to form finger 253 which engages the similar finger 242 of the thrust insert. The fingers provide an irrotational coupling between member 250 and the balloon tip, eliminating spurious twisting of the balloon. This allows the assembly to maintain precise rotational alignment of the balloon while axially extending it.

Member 250 may be formed of solid or of tubular stock; tubular stock advantageously permits the member to be advanced over a guide wire. Member 250 is preferably removed following balloon insertion for aortic fluid monitoring via the inner lumen. The metal stock used for forming the member 250 and the thrust insert, and the amount of material removed to form the respective (optional) engaging fingers, are selected so that the tube 214 holds the two end regions 242, 253 in firmly engaged alignment. For example, with tube 214 formed of 1.25mm inner diameter (ID) angioflex tubing, a thrust insert 240 was formed of 18 gauge thin wall steel tubing with a flat formed to a depth of .52 ± .025mm from its nominal 1.25mm outer diameter (OD). This left a .71 + .025mm thick protruding finger, or slightly over a semi-diameter of the tubular segment. The corresponding finger of the member 250 was formed of a half-diameter segment of 19 gauge regular wall steel tubing (i.e., .70 ID x 1.0mm OD) so that the .71mm thick finger 242 of the thrust insert and the .52mm thick finger 253 of the extender member 250 are pressed into firm engagement with each other.

The tubing 214 may itself be sufficiently stiff both to push and turn the balloon 204 when its proximal end is moved. Preferably, however, the tubing 214 is pliable and constitutes a relatively ineffective means for transmission of axial or torsional forces. The provision of the separate internal extender member 250 adds further stiffness which allows the use of appreciably softer tubing materials while providing for the delivery of small but precisely controlled motions to compact the balloon, substantially unperturbed by shear or compression along the length of the inner structure.

It will be appreciated that when firmer catheter or inner tubes are employed, the provision of an irrotational extending member is of less concern, and in that event the fork-like engaging fingers 242, 253 are not necessary. In that case, nosepiece 230 may be formed as a simple hub with a flat or concave thrust-receiving inner face.

Figure 3C shows details of the hub and thrust insert of such an embodiment. As shown, extender member 250 has a rounded nose 251, and the hub has a thrust receiving face 243 which member 250 is guided to bear against without any torque-transmissive coupling.

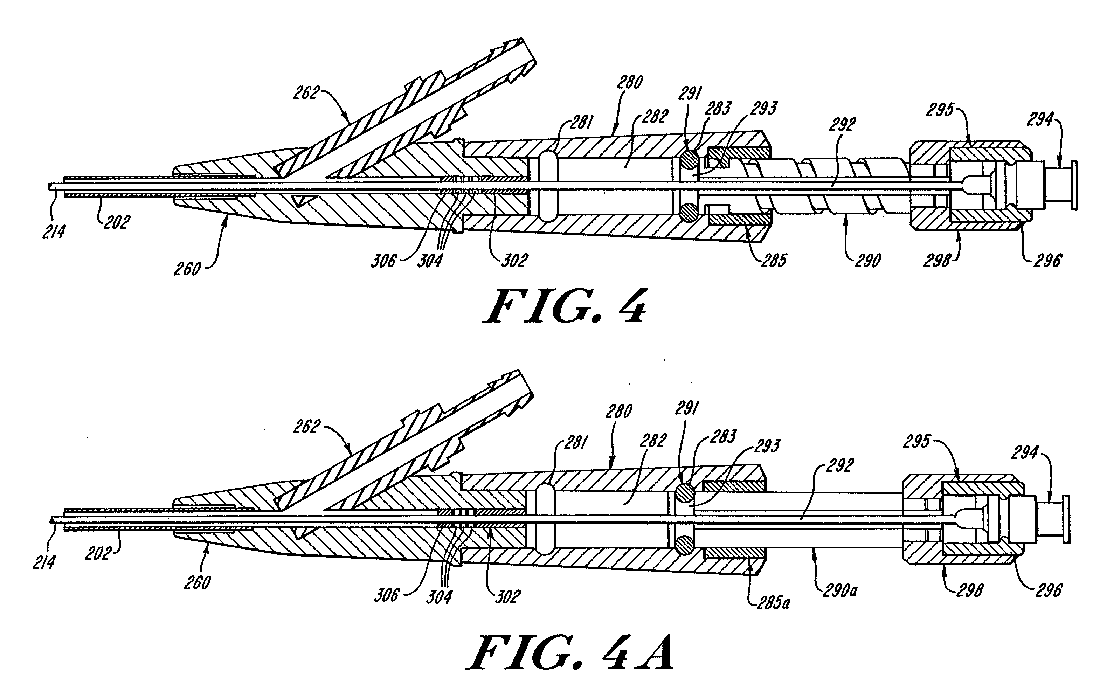

Figure 4 is a detailed cross-section of a handle portion 200 used with a thrust-twist extender as in Figures 2A and 2B. Handle 200 has a front portion 260 constituting a bifurcated manifold with an inflation port fitting 262 of conventional type communicating with the inflation tube 202, and an axial passage extending therethrough for holding the inner tube 214. A rear portion 280 constitutes an actuator housing having a central bore 282 axially aligned with the manifold 260 and cooperating with a male screw member 290 to move a blunt needle/tube assembly 292 back and forth. Tube 292 is attached at one end to a needle connector 294 which is fastened to screw member 290 by a means of a bushing 295, roller pin 296 and knurled knob 298.

The tube 292 extends through a first bushing 302, O-ring seals 304 and a second bushing 306 into the bifurcated manifold 260, where it is bonded to inner tube 214 (Figure 3) to provide a rigid coupling therewith. Needle connector 294 and tube 292 thus provide an access port for the inner, fluid-sampling tube 214.

The male screw mechanism 290 is cross-drilled at its tip, and a pair of small ball bearings 291 are held in the holes under tension by springs 293 to serve as detents. The spring loaded balls snap into grooves 281, 283, located at the advanced and retracted positions, respectively of the actuator housing 280. A threaded insert 285 bonded to the actuator housing 280 engages the male screw member.

The screw actuator thus serves to rotate and advance the inner tube 214 when the knurled knob 298 is turned. In use, the torquing member, 250 described with reference to Figure 3B above is inserted fully into tube 292 to engage the tip assembly of the balloon, and is locked in position by a Luer fitting at 294. Knob 298 is then turned to rotate and advance both the torquing member 250 and tube 214, with respect to the outer tube 202. Since the outer and inner tubes are connected to the proximal and distal ends of the balloon, this extends and twists the balloon by an amount equal to the motion of the screw.

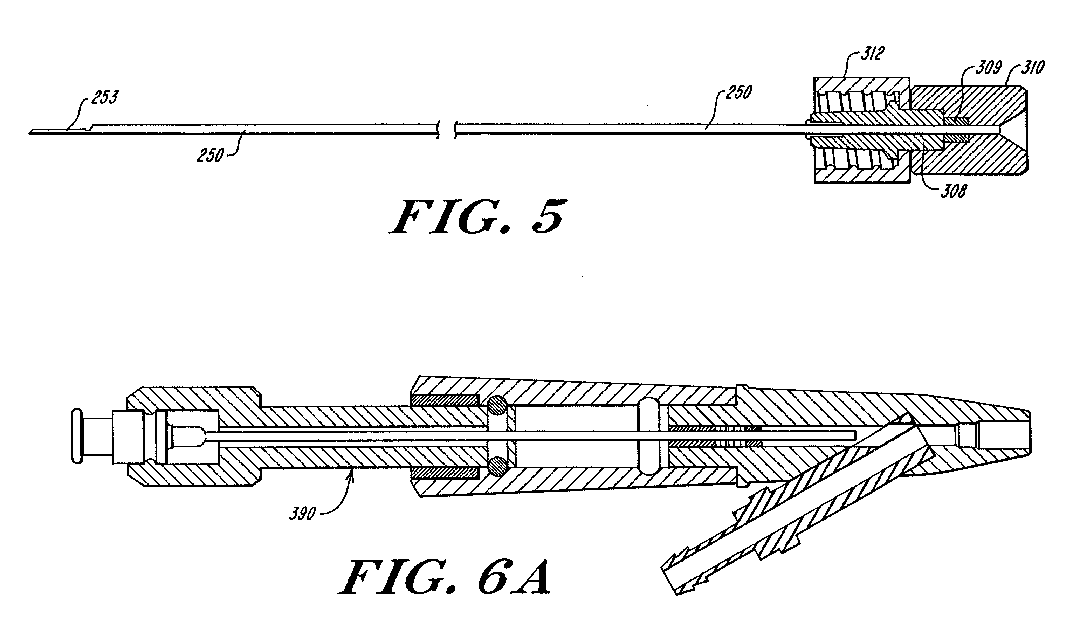

Figure 5 shows the torquing member 250, which is approximately one hundred twenty-five centimeters in length. The distal end is formed with the aforesaid engaging finger 253, and the proximal end is affixed to a cap 310 and Luer tapered plug 308 with a high friction locking adhesive 309. A lock nut 312 surrounds plug 308 for engaging the fitting 294 of the handle 200 (Figure 4) to lock the member 250 in the assembly.

The foregoing embodiments provide a screw mechanism which twist-wraps and stretches or extends a bladder or balloon at the end of a catheter, while still providing one or more lumens for balloon inflation and fluids monitoring. In the described preferred embodiment employing a steel tubular stiffening member, fluids may be monitored without withdrawing the stiffener from the handle.

Returning now to the alternate embodiment illustrated in Figures 2C, 2D which provides a twist-free purely extensional motion of defined magnitude to the balloon, Figure 4A shows a corresponding detailed cross-section of the handle portion 200 of the embodiment of Figures 2C, 2D.

Handle 200 has a front portion 260 constituting a bifurcated manifold with an inflation port fitting 262 of conventional type communicating with the inflation tube 202, and an axial passage extending therethrough for holding the inner tube 214. A rear portion 280 constitutes an actuator housing having a central bore 282 axially aligned with the manifold 260 and cooperating with an axially sliding member 290a to move a blunt needle/tube assembly 292 back and forth. Tube 292 is attached at one end to a needle connector 294 which is fastened to member 290a by a means of a bushing 295, roller pin 296 and knurled knob 298. Bushing 295 is cemented into knob 298, which, in-turn, retains the pin 296 in its hole to provide a rigid assembly.

The tube 292 extends through a first bushing 302, O-ring seals 304 and a second bushing 306 into the bifurcated manifold 260, where it is bonded to inner tube 214 (Figure 3) to provide a fluid-tight coupling therewith. Needle connector 294 and tube 292 thus provide an access port for the inner, fluid-sampling tube 214.

The axially movable member 290a is cross-drilled at its tip, and a pair of small ball bearings 291 are held in the holes under tension by springs 293 to serve as detents. The spring loaded balls snap into grooves 281, 283, located at the advanced and retracted positions, respectively of the actuator housing 280. An optional grooved insert 285a may be bonded to the actuator housing 280 to serve as an irrotational alignment bushing to engage a longitudinal guide rib or ridge formed on member 290a to prevent rotation of the assembly as it is moved.

The actuator thus serves to advance the inner tube 214 when the knurled knob 298 is advanced. In use, the extending member 250 described with reference to Figures 3B and illustrated in Figure 5, below, is inserted fully into tube 292 to engage the tip assembly of the balloon, and is locked in position by a Luer fitting at 294. Knob 298 is then pushed forward to advance both the extender member 250 and tube 214, with respect to the outer tube 202. Since the outer and inner tubes are connected to the proximal and distal ends of the balloon, their relative motion stretches the balloon by an amount equal to the motion of the knob 298, without twisting.

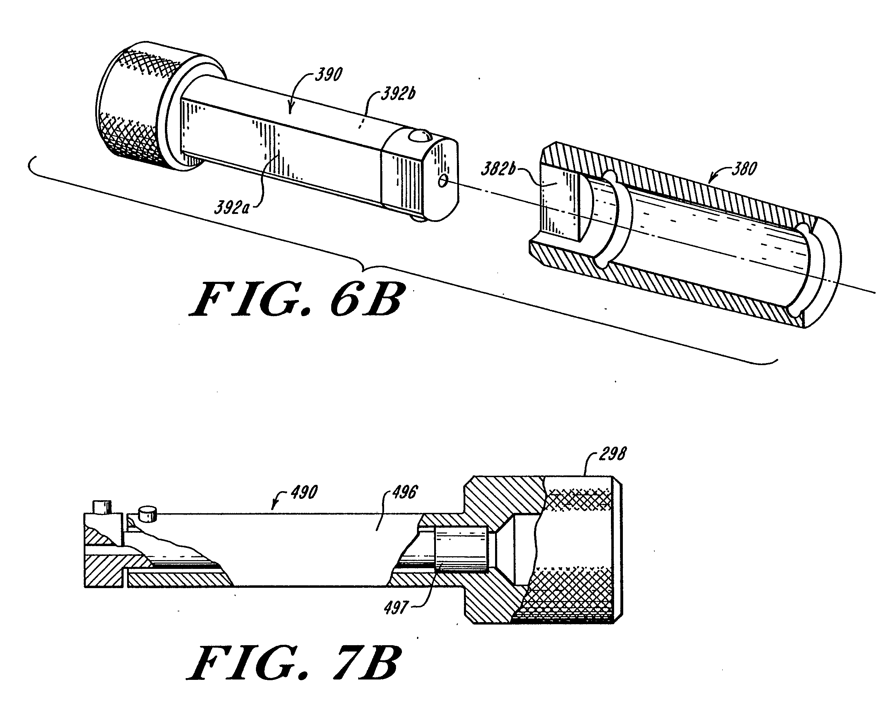

Figures 6A, 6B show a sectional and an exploded view of modified details of another embodiment of a stretch wrapping mechanism according to the invention. In this embodiment, the cooperating portions 390, 380 correspond to the portions 290, 280 just described, and the overall construction features are similar. A flat face 392b of the extender handle portion cooperates with a flat guide face 382b in the handle assembly to prevent turning of the inner assembly, i.e., the inner tube and extender member not shown).

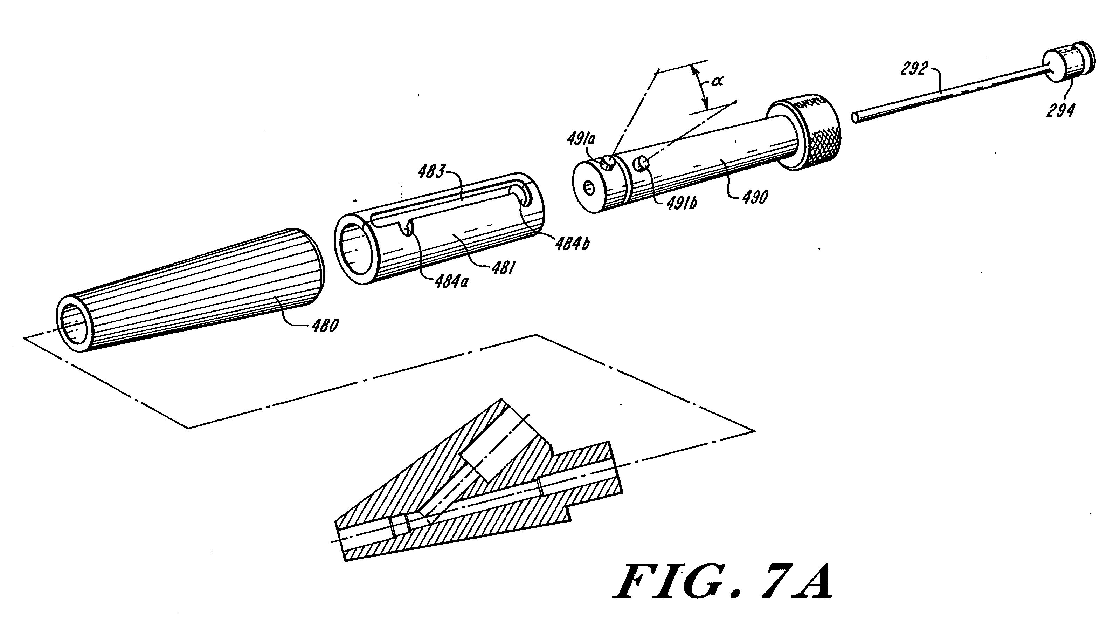

Figure 7A, 7B illustrate yet another embodiment of a balloon extension mechanism. In this embodiment, a rear portion 480 of the manifold handle receives a grooved split bushing 481 within which a lockable shank 490 moves back and forth. The shank assembly 490 holds a blunt needle assembly identical to the port assembly 292, 294 described with reference to Figure 4, which is attached to an inner tube 214, as previously described.

The split bushing 481 has an elongate groove 483 with laterally extending notches 484a, 484b, positioned in the manner of a locking drawbolt. The shank assembly 490 has a pair of protruding posts 491a, 491b, each of which has a width narrower than that of groove 483, but which are offset at an angle $\alpha$ such that the outer edges of the two posts are separated by a lateral distance greater than the groove width. Thus, as the shank 490 slides back and forth within bushing 481, the split bushing applies a gentle pressure against the posts forcing them into alignment with each other by elastic rotation of post shaft 495 relative to shank 490. Post shaft 495 is formed of a suitable elastic material and is secured to shank 490 at one end. When post 491b is aligned with a notch 484a or 484b, the shank rotates slipping the post into the notch and thereby stopping the shank at that longitudinal extension. A rotational force must then be exerted on the shank 490 in order to move post 491b out of the notch and permit the shank to move axially. This provides a positive detent or axial stop for the stretch assembly.

Figure 7B shows a further detail of the construction of the extender shank 490. As shown, an inner post shaft 495 bearing the groove-following post 491a is concentrically carried by, and cemented at its extreme end 497 to the outer shank sleeve 496. The outer shank sleeve 496 carries both the manipulation knob 298 and blunt needle assembly 294, 292, as well as the second, locking, post 491b. The inner sleeve 495 may be formed of an elastic material such as a hard rubber or soft plastic so that when the two posts are urged together by the groove walls the sleeve 495 twists slightly, providing a gentle torsional force which urges posts 491a, 491b apart. This provides the spring loading to snap post 491b into a notch.

The foregoing description illustrates various embodiments of a mechanism which stretches a bladder or balloon in a precise manner at the end of a catheter, while still providing one or more lumens for balloon inflation and fluids monitoring. As for the previously described embodiment, the twist-free steel tubular extender member permits a relatively soft IABP structure to be stiffened and straightened for insertion along an arterial path over a guide wire while still monitoring fluids through the balloon tip.

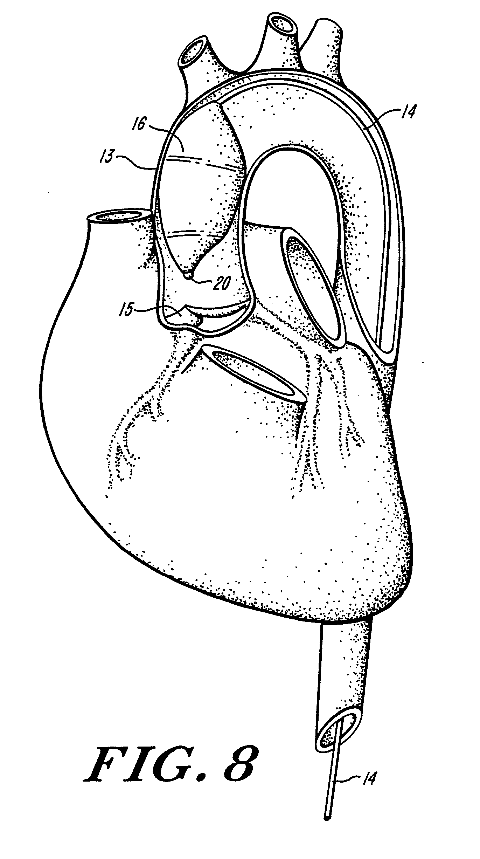

Returning now to the embodiment briefly discussed in relation to Figure 3, a more detailed description is made with reference to Figures 8-10 of a preferred balloon, of relatively large displacement, designed for counterpulsating pumping in a position close to the heart.

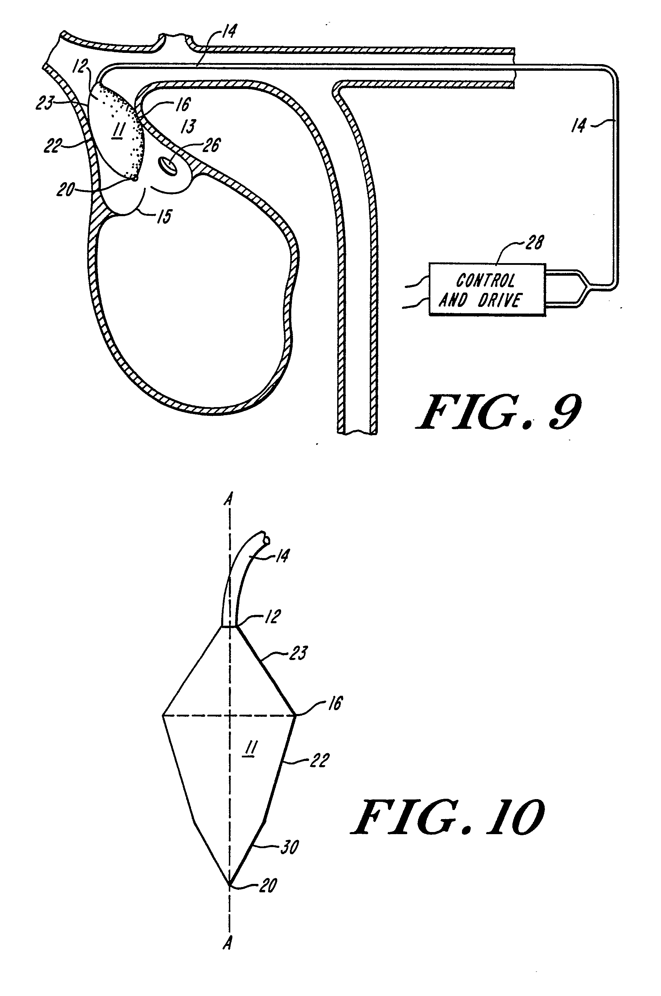

With reference to the figures a catheter balloon 11 is shown in its inflated condition positioned within an ascending aorta 13, just beyond the left ventricle valve 15. As shown in Figure 10, the balloon 11 has an elongated form along axis A-A with an orifice 12 for attachment to the catheter 14 to provide for inflation of the balloon. The balloon shape has a maximum periphery transverse to its long dimension at point 16 approximately midway between the ends of the balloon along the axis A-A. The balloon shape, when inflated, slopes outwardly from tip 20 at the end opposite to the orifice to this maximum point 16 along surface 22. The surface 23 of the balloon from the maximum dimension point 16 slopes inwardly to the orifice 12. A preferred material for this balloon is polyurethane elastomer, typically having a wall thickness of approximately 0.125 mm. Other suitable materials include latex and polyester.

In Figures 8 and 9 the balloon 11 is shown in its inflated condition within the aorta just outside of the left ventricle. As shown the widest periphery point 16 is a larger diameter than the aorta diameter and is therefore sufficiently large, upon inflation, to occlude the aorta. The balloon 11 is inserted in a collapsed condition at the end of the catheter 14 into the artery. A control and drive unit provides for inflation of the balloon to a pressure sufficient to occlude the artery after ventricular ejection. Upon inflation, the wider peripheral section at point 16 completes inflation first, blocking the aorta and the balloon then continues to inflate the roughly conical front section tapering down to tip 20. Under these circumstances, inflation of the balloon first blocks the artery and then increases the blood pressure within the volume of the artery between the heart valve 15 and the wide point 16 of the balloon. This increased pressure provides for increased blood flow to the coronary arteries 26. The tapers on the front portion of the balloon 30 and 22 are adjusted to provide for appropriate high pressure within the chamber formed by the valving action of the balloon and the heart valve 15. The extension of the wide transverse portion of the balloon to seal tightly against the aorta wall is accelerated with respect to inflation of the remainder of the balloon by the Bernoulli effect as the balloon is inflated.

The slope or taper running from the maximum width point 16 back to the orifice 12 is for the purpose of allowing the balloon in a collapsed condition to be small enough in cross section not to block blood flow through the artery. The degree of slope of this taper is determined in a practical fashion, depending upon the dimensions of the remainder of the balloon so as to provide the optimum collapsed profile.

In the typical configuration the portion of the balloon between the point of maximum diameter 16 and the forward end 20 has, in effect, two sections, the one nearer the midpoint having a shallower slope than that nearer the end. The shallower slope of the wall 22 is determined chiefly by the pressure and filling considerations discussed above to maximizes the pressure applied to the blood in the section of the aorta occluded upon inflation. The small foremost portion 30 having the steeper slope allows for a small collapsed profile at the tip end as does the taper section 23 at the orifice end.

While a specific embodiment of the invention have been described, it will be understood that variations in this shape may be accommodated within the scope of the invention.

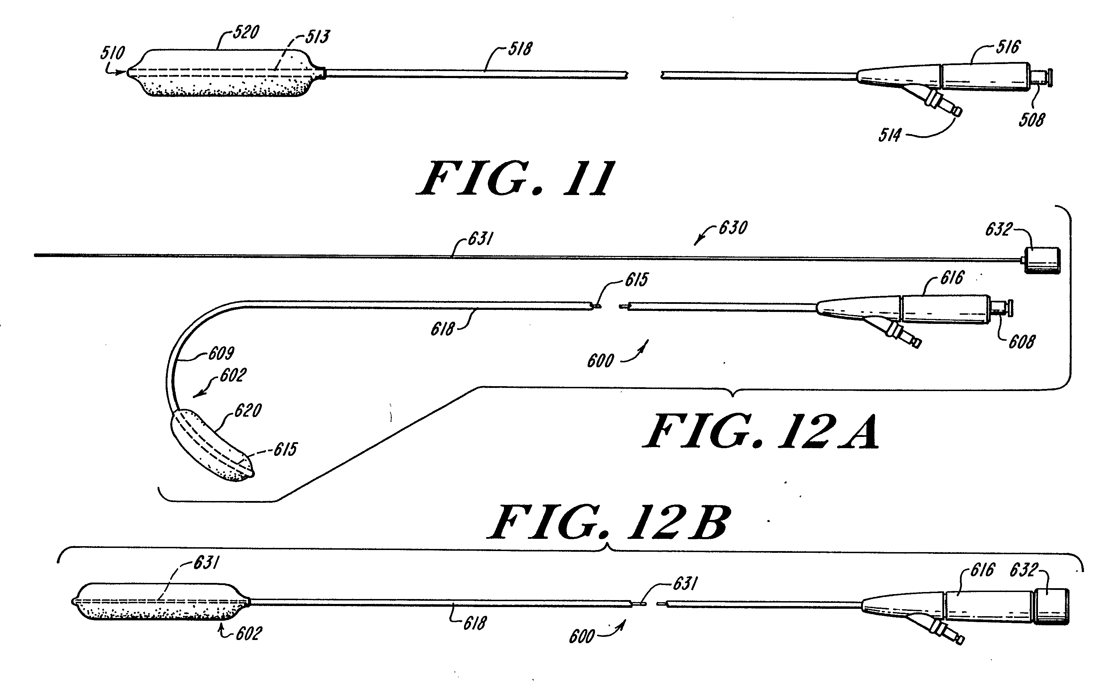

Insertion of the relatively large balloon to its pumping position past the aortic arch is effected, in accordance with another aspect of the invention, by providing an intially curved support member which may be selectively straightened during insertion, and which imparts a curvature to the balloon that allows the balloon to remain in a stable position as it undergoes pumping motion in the body. The structure and method of achieving these results will be described with reference to Figures 11-13.

By way of background, Figure 11 shows a prior art IABP which has a balloon 520 mounted on an inflation catheter 518 extending from a handle 516 which serves as a fluids manifold for connection to a pulsed source of inflation fluid, via port 514. Catheter 518 may carry an interior tube (not shown) which interconnects a fluid sampling port 510 at the distal end of the balloon with a second port 508 in the handle 516. In the prior art IABP the catheter 518 and an interior tube or balloon supporting member 513 each assume a straight profile in their relaxed state, although they are of sufficient flexibility to allow lateral bending for insertion as they follow a sheath or guide wire. It is also known in the art to provide a relatively stiff torquing wire or rod which extends from the handle to the balloon tip for twisting the balloon to compact it.

By contrast, the presently preferred embodiment of the invention disclosed herein, illustrated in Figure 12A, is an IABP assembly 600 wherein the balloon end 602 is formed with an intrinsic curvature in the region of the balloon 620 and preferably extending to the adjacent portion 609 of the inflation catheter 618. In Figure 12A, a tube 615 forming the inner lumen of the preferred embodiment is shown in phantom; the portion of tube 615 within the balloon serves to support the distal balloon end in longitudinal extension and prevent collapse, and thus constitutes a support structure. In other embodiments the balloon support structure may consist of a spiral wound wire tube or the like. According to the present invention, the balloon end is provided with a non-straight, and preferably curved balloon-supporting structure. A separate stiff straightening member 630, shown removed from the assembly, is adapted to fit within the catheter 618 for straightening the end during the initial stages of balloon insertion. The member 630 fits in through a central port, e.g., the fluid sampling port 608 in the illustrated two-lumen assembly, and its elongate solid or tubular stiffening rod 631 extends to the balloon tip.

Figure 12B shows the assembly of Figure 12A with the straightening member 630 fully inserted. For clarity of illustration, tube 615 is not shown, although it will be understood that in the embodiment under discussion stiffener 631 extends within, and is constrained by, that tube. The stiffness of stiffener 631 overcomes the intrinsic curvature of the different supporting members at the balloon end 602 of the device, and thus straightens the end. Knob 632 at the near end of member 630 locks to a mating fitting on the handle 616 by a bayonette mount, locking Luer fitting, or the like, to secure the member in position.

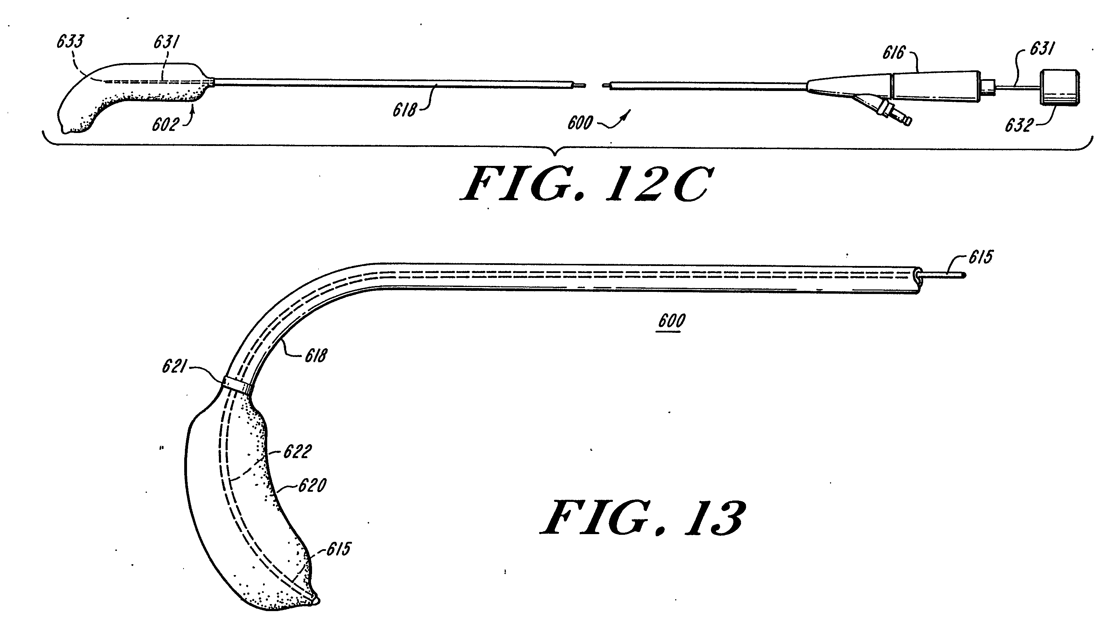

Figure 12C shows the assembly of Figure 12B with the straightening member 630 withdrawn a slight amount, about two to five centimeters, to allow the balloon end to partially re-assume its normal curvature. As shown, the end 633 of the straightening rod 631 is retracted from the balloon tip, and resides within the balloon. In this position, the balloon may be advanced around a sharply curved arterial path. When the rod 631 is withdrawn further, the balloon assembly reassumes its fully curved shape illustrated in Figure 12A, and may be advanced along a curved path of greater length.

In a preferred embodiment of the invention, a balloon is inserted into the ascending aorta by first straightening the assembly as shown in Figure 12B and advancing the catheter along an arterial path into the descending aorta, and then, when the balloon has advanced to the region of the aortic arch, simultaneously withdrawing the straightening member and advancing the curved balloon tip around the arch into the ascending aorta. Preferably the straightening member is withdrawn as the balloon is advanced, so that the tip portion of the assembly advances and curves around the arch simultaneously. This minimizes trauma to the aorta.

Figure 13 illustrates in greater detail the structure of a presently preferred embodiment of the balloon end of the assembly 600. As shown, a strong, curved envelope or balloon 620 is supported at one end by an inflation catheter 618 and at the other end by a fluid sampling tube 615 which extends within catheter 618. Balloon 620 is formed of a strong thin film, and tubes 618, 615 are formed by extrusion from a polyurethane material. In a prototype embodiment, the catheter tube and the support member tubes were formed by extrusion into bulk tubing from the polyurethane materials sold under the trade names "Pellethane" and "Isoplast", respectively, of Dow Chemical Company.

Each of the tubes 618, 615 was first heat-formed to produce at its end portion a radius of curvature of two to five centimeters in a region of the tubing extending over a distance of ten to twenty centimeters from the balloon distal end, and the two tubes were then aligned in a jig with their curvatures parallel. With the tubes held in this alignment, the balloon and handle were then mounted at opposed ends of the assembly. Suitable assembly techniques for attaching the balloon include solvent bonding, RF welding, heat welding, and adhesive or other bonding. During assembly, radio opaque markers are placed at the end of the catheter and on the support member, in a manner known in the art. This permits fluoroscopic visualization of the balloon and its direction of curvature during insertion. Preferably the envelope of balloon 620 was also formed having an intrinsic curvature between its ends matching that of the tubing. While not strictly cylindrical, the balloon ends were centered at the tubes, and in an unstressed state the balloon walls extended in a roughly curved or polygonal arch between the ends. Thus, "curvature", as applied to the balloon herein means simply following a generally curved path along the curved tubes in an unstressed state. The balloon shape was achieved by forming the envelope on appropriate mandrels.

A straightening member was then made up of nineteen gauge regular wall stainless steel tube stock. This stock was found to provide adequate torsional stiffness and resistance to lateral bending to both overcome the curvature of the catheter and support tubing and prevent twisting of the balloon. While a solid metal rod, preferably a wire under one millimeter diameter, could also be used, the tubing was preferred because it could be placed over a guide wire to facilitate insertion of the balloon assembly in a blood vessel, and, as noted above, would not impair functioning of the pressure lumen, permitting monitoring of pressure through the balloon tip.

The IABP assembly fabricated in this manner had an intrinsically curved end portion which would re-assume its curved shape when the straightening member was withdrawn. Not only was the IABP more readily insertable around the aortic arch, but the assembly required no steering wires and, since the straightener provided a high degree of stiffness, could employ soft tubing without impairing its ease of insertability.

This completes a description of the several aspects and preferred embodiments of the invention, and methods of practice, all of which have been described with general reference to double lumen IABP assemblies having a balloon dimensioned for insertion in the ascending aorta. It will be understood, however, that the invention comprehends other forms of insertable balloon apparatus, with differing balloons, intrinsic curvatures, number of lumens, and those having curved or uncurved lengths of adjacent catheter. For example, even a single-lumen device may be so formed.

The invention being thus disclosed, variations and modifications thereof will occur to those skilled in the art, and all such variations and modifications are considered to lie within the scope of the invention, as defined in the claims appended hereto.

## Claims

1. A balloon catheter assembly comprising
an inflation catheter having a proximal end connected to an inflation handle and also having a distal end,
a balloon having proximal and distal ends, said balloon proximal end being connected to said inflation catheter distal end,
a pressure lumen interconnecting said balloon distal end with said handle,
a thrust-receiving member non-rotatably secured to said balloon distal end, and
an extension member extending through the pressure lumen, one end thereof including means for irrotationally engaging said thrust receiving member, and the other end thereof extending from said balloon to said handle to permit stretching of the balloon without twisting as said other end is moved.

2. The balloon catheter assembly of claim 1, wherein the handle includes means for advancing the extension member while rotating it at a pitch greater than approximately five millimeters of advance per rotation.

3. The balloon catheter assembly of claim 1 wherein the extension member is hollow and does not occlude the pressure lumen.

4. The balloon catheter assembly of claim 1, wherein the extension member has a bending stiffness greater than that of said inflation catheter and pressure lumen.

5. The balloon catheter assembly of any of claims 1-4, wherein said extension member is a torsion resistant extension member and said handle includes mechanical means for precisely determining the angular alignment of said balloon distal end as said other end is moved.

6. The balloon catheter assembly of claim 5, wherein said extension means includes plural detents defining an advancing motion between (.15) and (.75) times the distance between said balloon distal end and said balloon proximal end.

7. The balloon catheter assembly of claim 6, wherein said extension member is insertable over a guide wire.

8. The balloon catheter assembly of claim 5, wherein the handle includes a port assembly communicating with said pressure lumen, and also includes a slide mechanism for sliding said port assembly back and forth in said handle, and wherein said extension member is insertable through said port assembly into said fluids sampling pressure lumen, and is removably lockable to the port assembly so that· motion of the slide mechanism causes equal motion of said extension member, said pressure lumen and said balloon distal end.

9. The balloon catheter assembly of claim 5, wherein said balloon includes a proximal end wall, a middle peripheral wall and a distal end wall, said walls together forming a closed envelope, and wherein a said end wall meets said middle peripheral wall at an obtuse angle.

10. The assembly of claim 9, wherein said balloon is non-symmetric.

11. An inflatable device for attachment to a catheter to be positioned in an aorta comprising,
a balloon formed of a thin elastomeric material, said balloon being generally elongate along a first axis and having its maximum inflated dimension in a plane approximately normal to said first axis at a point intermediate the ends of said balloon along said first axis, said balloon being provided with an orifice at one end for attachment to said catheter, said balloon tapering from said point of maximum dimension toward one of its ends to a dimension substantially reduced from the maximum dimension at that end, and tapering in the opposite direction toward the orifice end of said balloon with a generally symmetrical taper, said maximum inflated dimension being effective to occlude the ascending aorta.

12. A device in accordance with claim 11, wherein said balloon tapers in a generally symmetrical form.

13. A device in accordance with claim 11, wherein the slope of said balloon from said point of maximum dimension toward the closed or tip end of said balloon has plural substantially distinct values along the said first axis.

14. A device in accordance with claim 11, wherein the position along the first axis of the maximum dimension of said balloon is at substantially between halfway and the three quarters point between the ends of said balloon.

15. A device in accordance with claim 11, wherein the taper sections are asymmetrical so as to provide an off-axis orifice or off-axis tip section or both.

16. A device in accordance with any of claims 11-15, wherein said elongate first axis is curvilinear.

17. An insertable balloon assembly comprising a balloon having a first and second ends, a balloon supporting structure extending between the first and second ends of the balloon and having a curvature and stiffness effective to support said balloon in a curved shape, an inflation catheter, a handle assembly interconnected with the balloon by the inflation catheter, and a straightening member insertable over a guide wire through said catheter and having a bending stiffness effective to straighten said supporting structure.

18. An assembly according to claim 17, wherein said balloon has a curvature between opposed ends thereof effective to stabilize the balloon in a curved vessel during repetitive inflation and deflation cycles.

19. An assembly according to claim 17 or 18, wherein the straightening member is adapted for removable insertion through said catheter to straighten said supporting structure for enabling insertion of the balloon along a path, and said supporting structure conforms to the curvature of the aortic arch, so that by withdrawing the straightening member the balloon may advance past the aortic arch.

20. An insertable balloon assembly comprising a balloon having first and second ends,
a curved balloon support coupled to the first end of the balloon and extending through the balloon at least to the second end,
an inflation catheter coupled to the second end of the balloon and having a curvature substantially matching the balloon support at said second end,
a handle assembly connected to said catheter, and straightening means insertable through said handle, said catheter and said balloon support for straightening said balloon support so that the balloon may be inserted along an arterial passage, said straightening means having a bending stiffness greater than the bending stiffness of the curved balloon support, and said catheter, balloon support and straightening means together being sufficiently flexible for insertion to the aorta.

21. An insertable balloon assembly according to claim 20, wherein said support has a curvature conforming to the aortic arch.

# FIG. 1

*(PRIOR ART)*

# FIG. 2A

EP 0 363 203 A2

FIG. 2B

FIG. 2C

EP 0 363 203 A2

**FIG. 2D**

**FIG. 3**

EP 0 363 203 A2

*FIG. 3A*

*FIG. 3B*

*FIG. 3C*

FIG. 4

FIG. 4A

**FIG. 5**

**FIG. 6A**

EP 0 363 203 A2

FIG. 6B

FIG. 7B

EP 0 363 203 A2

FIG. 7A

FIG. 8

FIG. 9

FIG. 10

*FIG. 11*

*FIG. 12A*

*FIG. 12B*

EP 0 363 203 A2

FIG. 12C

FIG. 13

EP 0 363 203 A2